(19) Europäisches Patentamt
European Patent Office
Office européen des brevets

(11) Veröffentlichungsnummer : **0 469 480 A2**

# (12) EUROPÄISCHE PATENTANMELDUNG

(21) Anmeldenummer : **91112595.3**

(22) Anmeldetag : **26.07.91**

(51) Int. Cl.$^5$ : **C07D 309/30,** A61K 31/365

(30) Priorität : **01.08.90 DE 4024425**
**01.06.91 DE 4118009**

(43) Veröffentlichungstag der Anmeldung :
**05.02.92 Patentblatt 92/06**

(84) Benannte Vertragsstaaten :
**BE DE DK ES FR GB GR IT LU NL**

(71) Anmelder : **HOECHST AKTIENGESELLSCHAFT**
**Postfach 80 03 20**
**W-6230 Frankfurt am Main 80 (DE)**

(72) Erfinder : **Hammann, Peter, Dr.**
**Bürgermeister-Rühl-Strasse 20**
**W-6113 Babenhausen (DE)**
Erfinder : **Grabley, Susanne, Dr.**
**Hölderlinstrasse 7**
**W-6240 Königstein/Ts. (DE)**
Erfinder : **Granzer, Ernold, Dr.**
**Falkensteiner Strasse 24**
**W-6233 Kelkheim/Ts. (DE)**
Erfinder : **Romeyke, Yvonne**
**Im Antsee 2**
**W-6080 Gross-Gerau (DE)**

## (54) Verfahren zur stereoselektiven Herstellung von 5-substituierten delta-Lactonen und deren Verwendung.

(57) Es wird ein Verfahren zur stereoselektiven Herstellung von 5-substituierten δ-Lactonen der Formeln Ia, Ib, Ic und Id

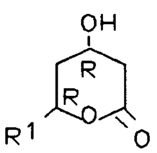 (Ia) 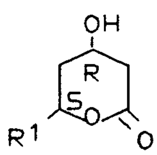 (Ib) 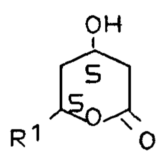 (Ic) 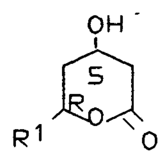 (Id)

wobei $R^1$ für geradkettige oder verzweigte Alkyl- oder Alkenylgruppe oder für Methylhydroxy steht, neue 5-substituierte δ-Lactone und neue Zwischenprodukte, sowie deren Verwendung als Arzneimittel mit Cholesterin-Synthese hemmender Wirkung, Duft- und Geschmackstoffe, beschrieben.

EP 0 469 480 A2

Die Erfindung betrifft Verfahren zur stereoselektiven Herstellung von 5-substituierten δ-Lactonen, neue 5-substituierte δ-Lactone und neue Zwischenprodukte, sowie deren Verwendung insbesondere als Arzneimittel mit Cholesterin-Synthese hemmender Wirkung, Duft- und Geschmacksstoffe.

Ein 5-substituiertes 3R5R δ-Lacton der Formel Ia

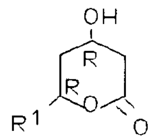

I a

wurde bereits enantioselektiv hergestellt, wobei $R^1$ für Pentyl steht (Bennet und Knight, Heterocycles 29, (1989), 639). Ausgangsverbindung war Methyl-3-oxo-5-hexenal, das mit Hilfe von Hefen asymmetrisch reduziert wurde. Die Ausbeute betrug 22 %, bei einer Enantiomerenreinheit von 76 %.

Aufgabe der vorliegenden Erfindung ist es ein Verfahren zu entwickeln mit dem alle Stereoisomere des δ-Lactons der Formel Ia in hoher Enantiomerenreinheit hergestellt werden können. Als Ausgangsmaterial wurde ein Streptenol der Formel

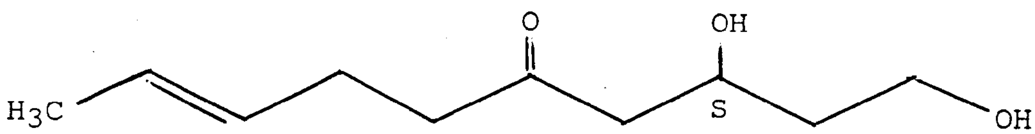

gewählt. Diese Verbindung wurde bereits beschrieben (Keller-Schierlein Helvetica Chimica Acta, 66, (1983), 1253) und läßt sich beispielsweise durch ein mikrobiologisches Verfahren durch Streptomyceten herstellen (EP-A 90 103 411.6; Mitzutani, J. Antibiotics, XLII (1989), 952).

Die Erfindung betrifft somit ein Verfahren zur Herstellung von 5-substituierten 3R5R, 3R5S, 3S5S und 3S5R δ-Lactonen der Formel Ia, Ib, Ic oder Id,

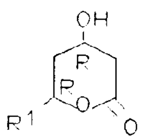 (Ia) 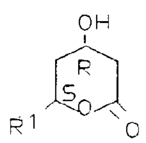 (Ib) 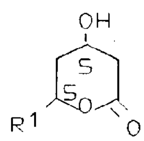 (Ic) 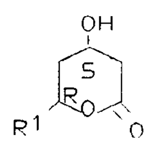 (Id)

wobei $R^1$ für

1) geradkettiges oder verzweigtes Alkyl mit 3 bis 15 C-Atomen,

2) geradkettiges oder verzweigtes Alkenyl mit 3 bis 15 C-Atomen und 1 bis 7 C-C-Doppelbindungen oder

3) $CH_2OH$ steht, dadurch gekennzeichnet, daß man

a) eine Verbindung der Formel IIa

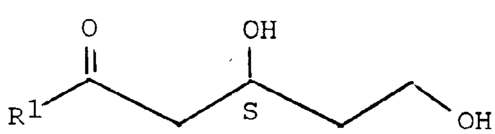

(IIa)

1) zu einer Verbindung der Formel IIIa oder IIIb,

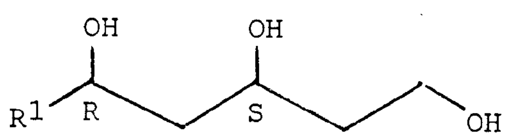

(IIIa)

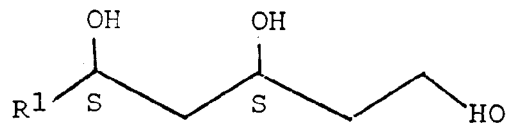

(IIIb)

diastereoselektiv reduziert

2) zu einer Verbindung der Formel Ia oder Ib,

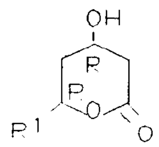

(Ia)

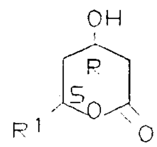

(Ib)

regioselektiv oxidiert, und gegebenenfalls

3) die Doppelbindungen der Alkylenkette hydriert, oder

b) die Verbindung der Formel IIa zu

1) einer Verbindung der Formel IVa oder IVb,

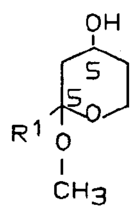

(IVa)

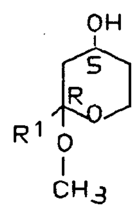

(IVb)

acetalisiert,

2) zu einer Verbindung der Formel V,

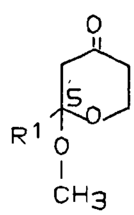

(V)

oxidiert,

3) zu einer Verbindung der Formel IVc,

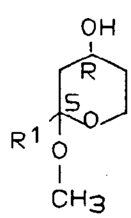

(IVc)

diastereoselektiv reduziert,

4) zu einer Verbindung der Formel IIb,

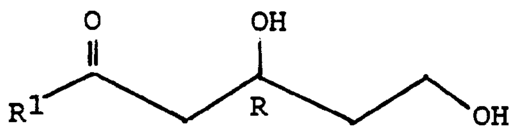

(IIb)

deacetalisiert,

5) zu einer Verbindung der Formel IIIc oder IIId,

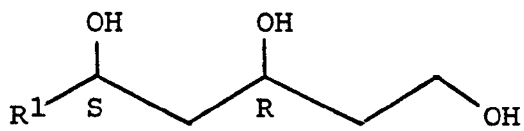

(IIIc)

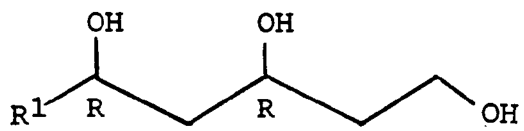

(IIId)

diastereoselektiv reduziert,

6) zu einer Verbindung der Formel Ic oder Id,

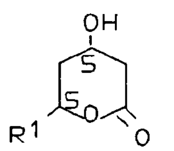

(Ic)

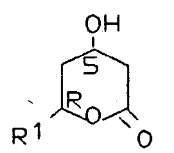

(Id)

regioselektiv oxidiert und gegebenenfalls

7) die Doppelbindungen in der Alkylenkette hydriert oder

c) die Verbindung der Formel Ia, Ib, Ic oder Id, wobei $R^1$ für

1) geradkettiges oder verzweigtes Alkyl mit 3 bis 15 C-Atomen

2) geradkettiges oder verzweigtes Alkenyl mit 3 bis 15 C-Atomen und 1 bis 7 C-C-Doppelbindungen steht,

1) mit einer Verbindung der Formel $R^3$-$O^-$ zu einer Verbindung der Formel

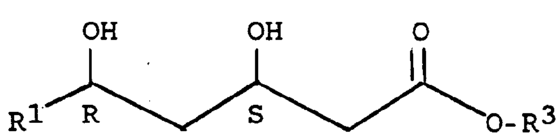

(Va)

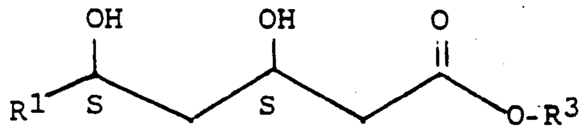

(Vb)

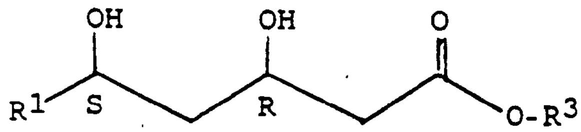

(Vc)

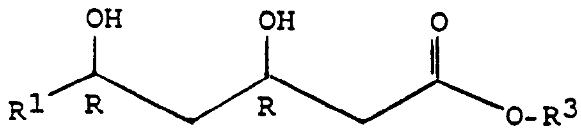

(Vd)

umsetzt, wobei $R^3$ für Alkyl mit 1 bis 5 C-Atomen oder tertiär-Butyl steht,

2) mit Aceton oder Dimethoxypropan zu einer Verbindung der Formel VIa, VIb, VIc oder VId ketalisiert,

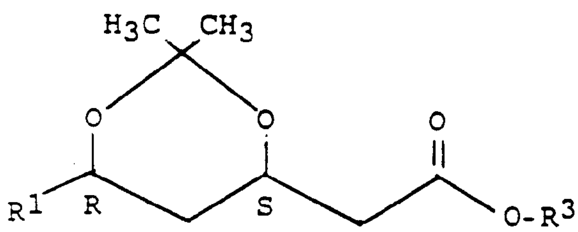

(VIa)

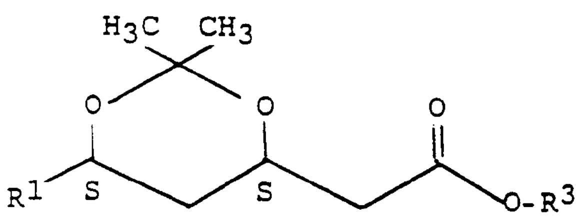

(VIb)

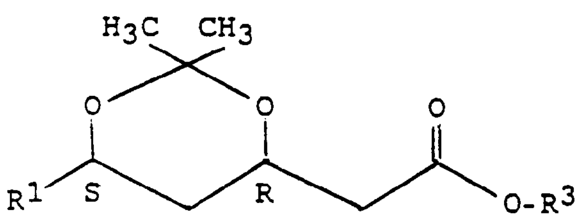

(VIc)

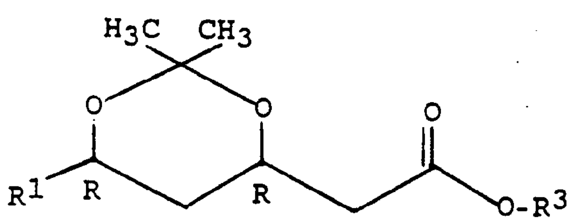

(VId)

3) mit Ozon zu einer Verbindung der Formel VIIa, VIIb, VIIc oder VIId,

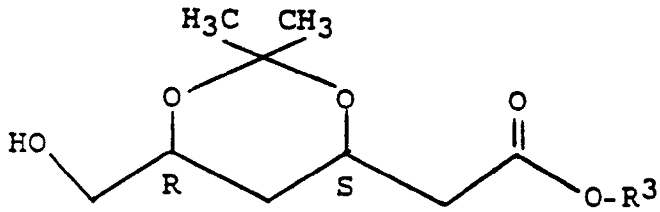

(VIIa)

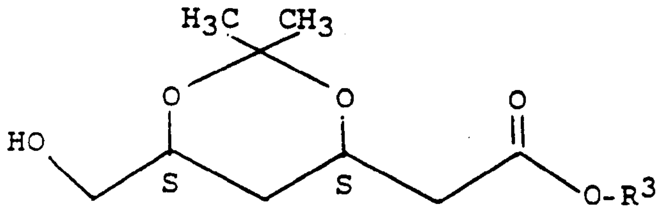

(VIIb)

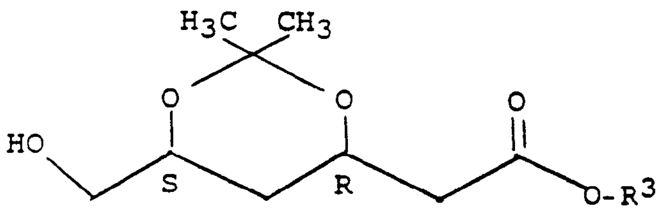

(VIIc)

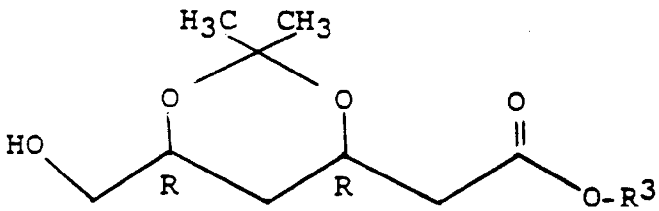

(VIId)

oxidiert und

4) zu einer Verbindung der Formel Ia, Ib, Ic oder Id umsetzt wobei $R^1$ für $CH_2OH$ steht.

Unter der Bezeichnung R und S wird die absolute Konfiguration am Kohlenstoffatom bezeichnet. R steht für rectus und S für sinister. Alle genannten Alkyl- und Alkenylreste mit mehr als 3 C-Atomen können sowohl geradkettig als auch verzweigt sein.

Gegenstand der Erfindung sind auch die Verbindungen der Formel Ia, Ib, Ic oder Id, ausgenommen die Verbindung Ia wobei $R^1$ für eine geradkettige Alkenylkette mit 5 C-Atomen steht.

Ein weiterer Gegenstand der Erfindung sind die Verbindungen der Formel IIb, IIIa, IIIc oder IIId, die sich als Zwischenprodukte eignen.

Gegenstand der Erfindung ist ferner ein Verfahren zur Herstellung der Verbindungen IIb, IIIa, IIIb, IIIc oder IIId.

Die Verbindungen der Formel VIIa, VIIb, VIIc oder VIId eignen sich als Vorprodukte zur Herstellung von Inhibitoren zur Cholesterin-Biosynthese (US 4,970,313).

Bevorzugt sind solche Verbindungen der Formel Ia bis Id bei denen R für Alkyl und Alkenyl mit 3 bis 10 C-Atomen steht und die Alkenylkette 1 bis 5 C-C-Doppelbindungen aufweist

Unter diesen Verbindungen sind wiederum jene der Formel Ia bis Id besonders hervorzuheben, in denen R für Alkyl und Alkenyl mit 5 C-Atomen steht und die Alkenylkette 1 oder 2 C-C-Doppelbindungen enthält.

Die zur Herstellung von Verbindungen der Formel Ia bis Id notwendige Verbindungen der Formel IIa können beispielsweise nach dem in der Europäischen Patentanmeldung Nr. 90 103 411.6 - auf die an dieser Stelle ausdrücklich Bezug genommen wird - vorgeschlagenen Verfahren hergestellt werden. Dabei wird die Verbindung der Formel IIa in einer Nährlösung, die eine Kohlenstoff-und eine Stickstoffquelle, sowie übliche anorganische Salze enthält, von Streptomyces spec., bevorzugt DSM 4356, produziert. Anstelle von Streptomyces spec. DSM 4356 können auch deren Mutanten und Varianten eingesetzt werden, soweit sie diese Verbindungen synthetisieren.

Die Bildung der Verbindung der Formel IIa verläuft besonders gut in einer Nährlösung, die Sojamehl und Mannit in Konzentration von jeweils 0,5 bis 6 %, bevorzugt 1 bis 4 %, enthält, bezogen auf das Gewicht der

gesamten Nährlösung.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35°C, vorzugsweise bei etwa 25 bis 30°C, insbesondere bei 28 bis 30°C erfolgen. Man kultiviert den Mikroorganismus unter den genannten Bedingungen bis die stationäre Phase erreicht ist, etwa 60 bis 120 Stunden, bevorzugt 70 bis 75 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Die Isolierung der Verbindung der Formel IIa (Streptenole) aus dem Kulturmedium erfolgt nach bekannten Methoden unter Berücksichtigung der chemischen, physikalischen und biologischen Eigenschaften der Produkte. Die Verbindung der Formel IIa liegt im Mycel bzw. in der Kulturbrühe vor. Sie können aüs der unfiltrierten Kulturbrühe mit einem mit Wasser nicht oder nur wenig mischbaren organischen Lösemittel, wie Chloroform oder Ethylacetat, extrahiert werden. Da sie sich jedoch nur zu einem geringen Teil im Mycel befinden, ist es vorteilhaft, die Kulturbrühe vom Mycel zu trennen, beispielsweise durch Zentrifugieren oder Filtrieren, vorzugsweise unter Zugabe von Filterhilfsmitteln. Die Verbindung der Formel IIa kann dann aus dem Überstand bzw. Filtrat isoliert werden, zweckmäßig im leicht sauren bis neutralen pH-Bereich, vorzugsweise bei pH 6 bis 7. Dazu kann man mit Wasser wenig oder nicht mischbare organische Lösemittel verwenden, insbesondere chlorierte Kohlenwasserstoffe, wie Chloroform oder Methylenchlorid oder Ester wie Ethylacetat oder Aceton.

Anstelle einer Extraktion können die Streptenole auch durch Adsorption an handelsüblichen Adsorberharzen aus der Kulturbrühe isoliert werden. Es hat sich ebenfalls als günstig erwiesen, den genannten Fermenterinhalt zu trocknen, beispielsweise durch Sprühtrocknen oder Gefriertrocknen.

Zur Isolierung der reinen Streptenole können die üblichen Verfahrensschritte Verwendung finden, wie Chromatographie oder Gelfiltration. Besonders bewährt hat sich eine Chromatographie an Kieselgel, wobei als Laufmittel eine Mischung aus Essigester und Hexan in einem Volumenverhältnis von beispielsweise 1:2 Verwendung findet.

Im folgenden werden die Verfahren a), b) und c) die es ermöglichen die 5-substituierten δ-Lactone der Formel Ia, Ib, Ic und Id stereoselektiv herzustellen, näher beschrieben.

Bei dem Verfahrensschritt a1 geht man am besten so vor, daß man die ß-Hydroxy-Keton-Gruppe der Verbindung IIa mit Lewissäure komplexiert und mit Alkoxydialkylboran, gegebenenfalls in einem inerten Lösungsmittel wie Diethylether oder Tetrahydrofuran (THF) reduziert und anschließend mit $NaBH_4$ zu einer Verbindung der Formel IIIa diastereoselektiv reduziert.

Bei der Herstellung der Verbindung der Formel IIIb geht man am besten so vor, daß man die Verbindung IIa in einem geeigneten Lösungsmittel, wie Acetonitril, Ether oder in Gemischen von Acetonitril mit Eisessig oder Ether mit Eisessig, mit $NaHB(OAc)_3$ oder $NH_4HB(OAc)_3$ stereoselektiv zu einer Verbindung der Formel IIIb reduziert.

Die Reaktionstemperaturen liegen dabei zwischen -70°C und +40°C, insbesondere zwischen -70°C und -20°C. Die Reaktionszeiten betragen 1 bis 10 Stunden, bevorzugt 2 bis 4 Stunden. Die Beendigung der Reaktion kann beispielsweise mittels Dünnschichtchromatographie bestimmt werden.

Die für den Verfahrensschritt notwendigen Reduktionsmittel sind, sofern nicht käuflich, auf einfache Weise nach literaturbekannten Verfahren herstellbar. So läßt sich beispielsweise Diethyl-methoxyboran aus Triethylboran und Methanol herstellen oder $NaHB(OAc)_3$ kann aus $NaBH_4$ und Eisessig hergestellt werden.

Bei dem Verfahrensschritt a2 geht man am besten so vor, daß man die Verbindungen IIIa oder IIIb aus Verfahrensschritt a1 mit einem Rutheniumkomplex (Murahashi, Tetrahedron Lett., 22, (1981), 1605), insbesondere mit $(PPh_3)_3RuCl_2$, in einem organischen Lösungsmittel wie $CHCl_3$, $CH_2Cl_2$, Aceton oder Benzol regioselektiv zum δ-Lacton der Formel Ia oder Ib oxidiert oder an einem Platin- oder Paladiumkatalysator in Gegenwart von Sauerstoff nach bekannten Methoden oxidiert. Die Reaktion kann mit äquimolaren Mengen oder gegebenenfalls in Gegenwart eines Oxidationsmittels, wie z.B. N-Methylmorpholin-N-oxid auch mit katalytischen Mengen des Rutheniumkomplexes durchgeführt werden.

Die Reaktionstemperaturen liegen dabei zwischen -70°C und +70°C, vorzugsweise bei Verwendung eines Lösungsmittels zwischen dem Festpunkt und dem Siedepunkt des Lösungsmittels, insbesondere zwischen -20°C und +30°C. Die Reaktiönszeiten betragen 5 bis 60 Stunden, bevorzugt 20 bis 40 Stunden. Die Beendigung der Reaktion kann z.B. mit Dünnschichtchromatographie bestimmt werden.

Bei dem Verfahrensschritt a3 geht man am besten so vor, daß man die Verbindungen Ia oder Ib mit geradkettigen oder verzweigten Alkenylrest gegebenenfalls in einem inerten Lösungsmittel wie Methanol, Ethanol,

Isopropanol oder Ethylacetat oder einem Gemisch dieser Lösungsmittel oder einem wäßrigen Gemisch dieser Lösungsmittel, in Gegenwart eines gängigen Hydrierkatalysators nach literaturbekannten Verfahren mit Wasserstoff zu den entsprechenden Verbindungen der Formel Ia oder Ib mit geradkettigen oder verzweigten Alkylrest umsetzt. Gängige Hydrierkatalysatoren sind beispielsweise Elemente der 8. Gruppe wie Platin, Palladium oder auch Nickel, die meist zum Zwecke der Vergrößerung der reaktiven Oberfläche, beispielsweise auf Aktivkohle-, Siliziumdioxid- oder Aluminiumoxidträgern aufgetragen sind.

Je nach verwendetem Katalysator kann die Reaktion sowohl ohne als auch mit Überdruck an Wasserstoff, beispielsweise bis zu 1 Atmosphäre, durchgeführt werden. Die Reaktionstemperaturen liegen zwischen 0°C und 40°C, vorzugsweise bei Raumtemperatur. Die Reaktionszeiten sind abhängig von der Ansatzgröße und der Konzentration der zu reduzierenden Verbindung.

Bei dem Verfahrensschritt b1 geht man am besten so vor, daß man die Verbindung IIa in äquimolaren Mengen oder in bis zu einem 50-fachen Überschuß von einem Alkohol wie beispielsweise Methanol, Ethanol oder Isopropanol in Gegenwart katalytischer Mengen einer Lewissäure zu einer Verbindung der Formel IVa und IV b umgesetzt. Die Reaktion kann gegebenenfalls auch in einem inerten Lösungsmittel wie Chloroform, Methylenchlorid, THF, Essigester oder Dioxan durchgeführt werden. Als Lewissäure eignen sich beispielsweise Kupfer-, Eisen- oder Lithium-Halogenide, insbesondere $CuCl_2$, $FeCl_3$ oder LiBr.

Die Konzentration der Lewissäure - bezogen auf die Verbindung der Formel IIa - beträgt 0,1 bis 5 Gew.-%, bevorzugt 0,1 bis 1 Gew.-%. Die Reaktionstemperaturen liegen dabei zwischen -40°C und +100°C, insbesondere zwischen 0°C und 30°C, vorzugsweise bei Verwendung eines Lösungsmittels zwischen dem Festpunkt und dem Siedepunkt des Lösungsmittels, insbesondere zwischen,0°C und 30°C. Die Reaktionszeiten betragen 1 bis 180 Minuten, bevorzugt 5 bis 60 Minuten. Die Beendigung der Reaktion kann beispielsweise mittels Dünnschichtchromatographie bestimmt werden.

Bei dem Verfahrensschritt b2 geht man am besten so vor, daß man die Verbindung der Formel IVa in äquimolaren Mengen oder in bis zu einem 50-fachen Überschuß, gegebenenfalls in einem inerten Lösungsmittel wie Chloroform, Tetrachlorkohlenstoff, Methylenchlorid oder Hexan mit einem Oxidationsmittel wie Pyridiniumchlorochromat, Pyridiniumdichromat, Tetrapropylammoniumruthenat oder DMSO in Gegenwart von Acetanhydrid, Oxalylchlorid oder Trifluoressigsäureanhydrid bis zur Beendigung der Reaktion umsetzt.

Die Reaktionstemperaturen liegen dabei zwischen -70°C und +100°C, vorzugsweise bei Verwendung eines Lösungsmittels zwischen dem Festpunkt und dem Siedepunkt des Lösungsmittels, insbesonder zwischen -70°C und +40°C. Die Reaktionszeiten betragen 1 bis 180 Stunden, bevorzugt 1 bis 48 Stunden, besonders bevorzugt 1 bis 28 Stunden. Die Beendigung der Reaktion kann beispielsweise mittels Dünnschichtchromatographie (DC) bestimmt werden.

Die Oxidationsmittel für den Verfahrensschritt b2 sind käuflich.

Bei dem Verfahrensschritt b3 geht man am besten so vor,daß man die Verbindung der Formel V aus Verfahrensschritt b2 mit einem Alkali- oder Erdalkaliborat, beispielsweise mit $NaBH_4$ in einem Lösungsmittel, bevorzugt in einem Alkohol, wie Methanol, Ethanol oder Isopropanol, oder in einem Ether wie Tetrahydrofuran, stereoselektiv reduziert. Besonders bevorzugte Reduktionsmittel sind Alkylboranate, beispielsweise ®LS-Selectride (Aldrich, Steinheim, BRD).

Die Reaktionstemperaturen liegen dabei zwischen -70°C und +100°C, vorzugsweise zwischen dem Festpunkt und dem Siedepunkt des Lösungsmittels, insbesondere zwischen -70°C und +10°C. Die Reaktionszeiten betragen 1 bis 60 Stunden, bevörzugt 5 bis 20 Stunden. Die Beendigung der Reaktion kann beispielsweise mittels DC-Kontrolle bestimmt werden.

Bei dem Verfahrensschritt b4 geht man am besten so vor, daß die Verbindung der Formel IVc in äquimolaren Mengen oder in bis zu einem 50-fachen Überschuß von einem , Alkohol-Wasser-Gemisch, insbesondere einem Isopropanol-Wasser-Gemisch, gegebenenfalls in einem inerten Lösungsmittel wie Chloroform, Methylenchlorid, THF, Essigester oder Dioxan, in Gegenwart katalytischer Mengen einer Lewissäure bis zur Beendigung der,Reaktion umgesetzt wird. Als Lewissäure eignen sich beispielsweise Kupfer-, Eisen-oder Lithium-Halogenide, insbesondere $CuCl_2$, $FeCl_3$ oder LiBr.

Die konzentration der Lewissäure - bezogen auf die Verbindung der Formel IVc - beträgt 0,1 bis 5 Gew.-%, bevorzugt 0,1 bis 1 Gew.-%. Die Reaktionstemperaturen liegen dabei zwischen -40°C und +100°C, insbesondere zwischen 0°C und 30°C, vorzugsweise bei Verwendung eines Lösungsmittels zwischen dem Festpunkt und dem Siedepunkt des Lösungsmittels, insbesondere zwischen 0°C und 30°C. Die Reaktionszeiten betragen 1 bis 180 Minuten, bevorzugt 5 bis 60 Minuten. Die Beendigung der Reaktion kann beispielweise mittels Dünnschichtchromatographie bestimmt werden.

Bei dem Verfahrensschritt b5 geht man am besten in analoger Weise wie beim Verfahrensschritt a1 vor. Die Verbindung der Formel IIb wird mit einem Alkoxydialkyboran und $NaBH_4$ zu einer Verbindung der Formel IIIc diastereoselektiv reduziert oder mit $NaHB(OAc)_3$ oder $NH_4HB(OAc)_3$ zu einer Verbindung der Formel IIId diastereoselektiv reduziert.

Bei dem Verfahrensschritt b6 geht man am besten in analoger Weise wie beim Verfahrensschritt a2 vor. Die Verbindung der Formel IIIc oder IIId werden in Gegenwart eines Rutheniumkomplexes regioselektiv zum δ-Lacton der Formel Ic oder Id oxidiert.

Bei dem Verfahrensschritt b7 geht man am besten in analoger Weise wie beim Verfahrensschritt a3 vor. Die Verbindungen Ic oder Id mit geradkettigem oder verzweigtem Alkenylrest werden wie in Verfahrensschritt a3 beschrieben mit Wasserstoff zu den entsprechenden Verbindungen der Formel Ic oder Id mit geradkettigen oder verzweigten Alkylrest umgesetzt.

Bei dem Verfahrensschritt c1) geht man am besten so vor, daß man ausgehend von den Verbindungen Ia, Ib, Ic oder Id, wobei $R^1$ für geradkettiges oder verzweigtes Alkyl oder Alkenyl mit 3 bis 15 C-Atomen steht, mit einem Alkohol in Anwesenheit von Natriumhydrid zur entsprechenden Verbindung Va, Vb, Vc oder Vd umestert.

Die Reaktionstemperaturen liegen dabei zwischen dem Festpunkt und dem Siedepunkt des Lösungsmittels, insbesondere zwischen -20°C und +30°C. Die Reaktionszeiten liegen zwischen 3 und 10 Stunden. Die Beendigung der Reaktion kanr z.B. mit Dünnschichtchromatographie (DC) bestimmt werden.

Bei dem Verfahrensschritt c2) geht man am besten so vor, daß man die Verbindungen Va, Vb, Vc oder Vd aus Verfahrensschritt 1 mit Aceton oder Dimethoxypropan in Anwesenheit von Zinkchlorid zu den Verbindungen VIa, VIb, VIc oder VId ketalysiert.

Die Reaktionstemperaturen liegen dabei zwischen 20°C und 70°C. Die Reaktionszeiten betragen 5 bis 60 Stunden, bevorzugt 8 bis 20 Stunden.

Bei dem Verfahrensschritt c3) wird die Alkylseitenkette $R^1$ der Verbindungen VIa, VIb, VIc und VId durch Ozonolyse und Zugabe von Natriumborhydrid zur Methylhydroxygruppe verkürzt.

Die Ozonolyse erfolgt nach bekannten Methoden (Organikum, 14. Auflage, 1975, Seite 295, VEB-Verlag).

Die Verbindungen der Formel Ia, Ib, Ic oder Id, wobei $R^1$ für Methylhydroxygruppe steht, erhält man aus den entsprechenden Verbindungen VIIa, VIIb, VIIc und VIId durch saure Abspaltung der Schutzgruppen. Dies erfolgt beispielsweise mit Trifluoressigsäure in einem inerten Lösungsmittel.

Die Kettenverlängerung der Alkylseitenkette $R^1$ erfolgt ausgehend von der Verbindung der Formel IIa nach literaturbekannten Verfahren. Die Kettenverlängerung erfolgt beispielsweise durch Ozonolyse zum Aldehyd und anschließender Carbonyl-Olefinierung z.B. nach Wittig (Organikum, VEB-Verlag, 1975, Seite 295 und 434).

Die Reinigung, Isolierung und Aufarbeitung der Substanzen erfolgt nach den üblichen Methoden; beispielsweise können die Reaktionsprodukte durch Chromatographie an polaren Trägermaterialien wie Kieselgel oder ®Sephadex LH 20 mit Lösungsmitteln wie niederen Alkanolen wie Methanol, Chloroform, Dichlormethan oder Essigester oder Methanol/ Chloroform-Mischungen oder Essigester/Hexan-Mischungen aber auch durch extraktive Methoden wie flüssig/flüssig-Extraktion oder fest/flüssig-Extraktion gereinigt werden.

Die Verbindungen der Formel I und deren physiologisch verträgliche Salze eignen sich aufgrund ihrer wertvollen pharmakologischen Eigenschaften sehr gut zur Anwendung als Heilmittel.

Erfindungsgegenstand sind daher auch Arzneimittel mit einem Gehalt an mindestens einer Verbindung der Formel Ia, Ib, Ic oder Id. Die Arzneimittel eignen sich vorzugsweise zur Prophylaxe und/oder Therapie von Störungen des Stoffwechsels durch Cholesterin und cholesterinähnlichen Stoffen.

Die erfindungsgemäßen Arzneimittel werden im allgemeinen oral oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polethylenglykole und Lösungsmittel, wie etwa steriles Wasser, Alkohole, Glycerin und mehrwertige Alkohole genannt.

Vorzugsweise werden die pharmazeutischen Präparate in Dosierungseinheiten hergestellt und verabreicht, wobei jede Einheit als aktiven Bestandteil eine bestimmte Dosis mindestens einer Verbindung der Formel Ia, Ib, Ic oder Id enthält. Bei festen Dosierungseinheiten wie Tabletten, Kapseln und Suppositorien, kann diese Dosis bis zu etwa 500 mg, bevorzugt jedoch etwa 50 bis 300 mg, und bei Injektionslösungen in Ampullenform bis zu etwa 150 mg, vorzugsweise aber etwa 10 bis 100 mg, betragen.

Für die Behandlung eines erwachsenen Patienten sind - je nach Wirksamkeit der Verbindungen gemäß Formel Ia, Ib, Ic oder Id am Menschen - Tagesdosen von etwa 20 bis 500 mg Wirkstoff, vorzugsweise etwa 50 bis 300 mg, bei oraler Verabreichung und von etwa 5 bis 300 mg, bevorzügt etwa 10 bis 100 mg, bei intravenöser Applikation indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Die erfindungsgemäßen Arzneimittel werden dadurch hergestellt, daß man mindestens eine Verbindung der Formel Ia, Ib, Ic oder Id mit üblichen Träger- sowie gegebenenfalls Zusatz- und/oder Hilfsstoffen in die bzw. eine geeignete Darreichungsform bringt.

Die Verbindung der Formel Ia zeigt hervorragende Wirkung als Inhibitor der Cholesterin-Biosynthese. Sie kann dementsprechend als Lipidsenker eingesetzt werden. Aufgrund ihrer pharmakologischen Eigenschaften eignet sich die Verbindung der Formel Ia zur Behandlung und Prophylaxe von Störungen des Stoffwechsels durch Cholesterin und cholesterinähnlichen Stoffen.

Ferner eignen sich die Verbindungen der Formel Ia, Ib, Ic und Id als Aroma- und Duftstoffe.

Nachfolgend ist die Erfindung an Hand von Beispielen näher erläutert.

Alle nach den folgenden Beispielen erhaltenen Verbindungen wurden mittels $^1$H-NMR und/oder $^{13}$C-NMR und/oder IR und/oder C,H-Analyse und/oder Massenspektrum charakterisiert.

Herstellung der Verbindung der Formel IIa

a) Herstellung einer Sporensuspension des Produzentenstamms DSM 4356: 100 ml Nährlösung (4 g Hefeextrakt, 10 g Malzextrakt, 4 g Glucose, 1 l Leitungswasser, pH-Wert vor dem Sterilisieren 7,3) in einem 500 ml Erlenmeyerkolben werden mit dem Stamm beimpft und 72 Stunden bei 27°C und 120 UpM auf der rotierenden Schüttelmaschine inkubiert. Anschließend werden 20 ml Kulturflüssigkeit in einem 500 ml Erlenmeyerkolben mit dem Nährboden der oben genannten Zusammenstzung, dem 20 g Agar/l zur Verfestigung zugegeben wurde, gleichmäßig verteilt und dekantiert. Die Kulturen werden 10 bis 14 Tage bei 27°C inkubiert. Die nach dieser Zeit entstandenen Sporen eines Kolbens werden mit 500 ml entionisiertem Wasser, das einen Tropfen eines handelsüblichen nichtionischen Tensids enthält, abgeschwemmt, sofort weiterverwendet oder bei -22°C aufbewahrt.

b) Herstellung einer Kultur bzw. Vorkultur eines Produzentenstammes im Erlenmeyerkolben:

Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 % (pH 7.2 vor dem Autoklavieren) wird mit einer auf einem Schrägröhrchen gezogenen Kultur oder mit 0,2 ml Sporensuspension angeimpft und auf einer Schüttelmaschine bei 120 UpM und 27°C inkubiert. Die maximale Produktion des gewünschten Stoffes ist nach 72 Stunden erreicht. 10 und 100 l Fermenter werden 5 %ig mit einer 48 Stunden alten Submerskultur aus der gleichen Nährlösung angeimpft.

c) Herstellung der Verbindung der Formel IIa:

Ein 10 l Fermenter wird unter folgenden Bedingungen betrieben:

| | |
|---|---|
| Nährmedium: | 2 % Mannit |
| | 2 % Sojamehl |
| | pH 7,2 |
| Inkubationszeit: | 72 Stunden |
| Inkubationstemperatur: | 30°C |
| Rührergeschwindigkeit: | 250 UpM |
| Belüftung: | 4 l Luft/Min. |

Durch wiederholte Zugabe weniger Tropfen ethanolischer Polyollösung kann die Schaumentwicklung unterdrückt werden. Das Prodüktionsmaximum wird nach ca. 70 Stunden (pH = 5,3) erreicht. Die Ausbeuten liegen bei ca. 20 mg/l.

d) Isolierung der Verbindung der Formel II

Nach der Fermentation der Produzentenstämme wird die Kulturbrühe unter Zusatz von 2 % Celite als Filterhilfsmittel filtriert. Das Mycel wird mit Ethylacetat extrahiert und die organische Phase eingedampft. Das Kulturfiltrat wird getrocknet und der Rückstand mit Essigester extrahiert. Das Rohprodukt wird an einer Kieselgelsäule (Kieselgel 60, Macherey-Nagel) mit Essigester/Hexan (1:2, v:v) chromatographiert.

Beispiel 1

(+)-(3S,5R,8E)-1,3,5-Trihydroxy-8-decen (Verbindung 3a)

1 g (5,4 mmol) der Verbindung der Formel IIa, wobei $R^1$ für 2-penten steht, wird in 50 ml Tetrahydrofuran mit 8,6 ml einer 1 molaren Triethylboranlösung in Tetrahydrofuran (8,6 mmol) 15 min bei Raumtemperatur gerührt. Nach Abkühlen auf -70°C werden 4,3 ml Methanol sowie 0,4 g (10,7 mmol) Natriumborhydrid hinzugefügt und die Reaktionsmischung wetere 3 h bei dieser Temperatur gerührt. Zur Aufarbeitung werden 30 ml einer gesättigten

Natriumhydrogencarbonatlösung dazugegeben und dreimal mit jeweils 100 ml Essigsäureethylester extrahiert. Die Esterphase wird über Natriumsulfat getrocknet. Nach Abfiltrieren entfernt man das Lösemittel durch Destillation im Vakuum und erhält ein öliges Produkt. Dieses wird noch fünfmal mit Methanol versetzt und im Vakuum destilliert, so daß überschüssiges Boran entfernt wird. Der verbleibende Rückstand wird an Kieselgel mit Aceton:Hexan (2:3) gereinigt und ergibt die Verbindung 3a in einer Ausbeute von 0,82 g (81 %) als ein farbloses Öl.

Rf = 0,40 ($CH_2Cl_2$:$CH_3OH$/9:1); $[\alpha]^D_{20}$ = +4,4° (c=1, $CH_2Cl_2$);
Analyse für $C_{10}H_{20}O_3$ (188,27): berechnet: C 63,8; H 10,7;
gefunden: C 63,9; H 10,7;
$^1$H-NMR (400 MHz) δ = 1,5 - 1,7 (m, H-2, H-4, H-6, H-10); 2,1 (m, 2H, H-7); 3,75 (m, 3H, H-1, H-5); 4,1 (m, 1H, H-3); 5,45 (m, 2H, H-8, H-9);
$^{13}$C-NMR (100 MHz) δ = 17,8 (C-10); 28,4 (C-7); 37,7 (C-6); 39,0 (C-2); 43,0 (C-4); 60,4 (C-1); 71,5 (C-5); 72,0 (C-3); 125,35 (C-9); 130,7 (C-8);
IR ($CHCl_3$) m = 3200 - 3600 (OH).

Beispiel 2

(+)-(3S,5S,8E)-1,3,5-Trihydroxy-8-decen (Verbindung 3b)

Zu einer Suspension von 900 mg (23,8 mmol) Natriumborhydrid in 25 ml Tetrahydrofuran werden bei -70°C während 10 min 4,3 ml Eisessig zugetropft und 2 h bei dieser Temperatur gerührt. Nach Zugabe von 1 g (5,4 mmol) der Verbindung der Formel IIa, wobei $R^1$ für 2-Penten steht, in 5 ml Tetrahydrofuran werden noch 20 ml Eisessig während 30 min zugetropft und die Reaktionsmischung noch 2 h bei dieser Temperatur gerührt.

Nach vollständigem Umsatz kontrolliert durch DC wird das Gemisch mit 10 ml Wasser und 30 ml gesättigter Natriumhydrogencarbonatlösung versetzt und dreimal mit je 100 ml Ethylacetat extrahiert. Die organische Phase wird noch dreimal mit je 50 ml Wasser gewaschen und anschließend über Natriumsulfat getrocknet. Nach Entfernen des Lösungsmittels durch Destillation im Vakuum wird der Rückstand an Kieselgel mit Aceton:Hexan (2:3) chromatographisch gereinigt und ergibt die Verbindung 3b zu 78 % (0,8 g).

Rf = 0,37 ($CH_2Cl_2$:$CH_3OH$/9:1); $[\alpha]^D_{20}$ = +9,5° (c=1, $CH_2Cl_2$);
Analyse für $C_{10}H_{20}O_3$ (188,27): ber.: C 63,8; H 10,7;
gefunden: C 63,7; H 10,5;
$^1$H-NMR (400 MHz) δ = 1,65 - 1,85 (m, 9H, H-2, H-4, H-6, H-10); 2,1 (m, 2H, H-7); 3,9 (m, 3H, H-1); 4,0 (m, 1H, H-5); 4,2(m, 1H, H-3); 5,45 (m, 2H, H-8, H-9);
$^{13}$C-NMR (100 MHz) δ = 17,9 (C-10); 29,0 (C-7); 37,0 (C-6); 38,3 (C-2); 42,6 (C-4); 61,9 (C-1); 69,15 (C-5); 69,8 (C-3); 125,6 (C-9); 130,7 (C-8);
IR ($CHCl_3$) m = 3200 - 3600,(OH).

Beispiel 3

(+)-(3R,5R,8E)-3-hydroxy-5-dec-8-enolid (Verbindung 1a)

300 mg (1,59 mmol) der Verbindung 3a nach Beispiel 1 werden in 15 ml Benzol gelöst und mit 1,39 g (1,59 mmol) Tris-(triphenylphosphin)-ruthenium(II)-chlorid versetzt. Das Gemisch wird 3 Tage bei Raumtemperatur gerührt. Nach Entfernen des Benzols durch Destillation im Vakuum wird der teerige schwarze Rückstand in 2 ml Aceton gelöst und an Kieselgel mit Essigsäureethylester/Hexan (1:1) gereinigt. Die Verbindung entsteht als farbloses Öl in 68 %iger Ausbeute (200 mg).

Rf = 0,51 (Ethylacetat:Hexan/3:1); $[\alpha]^D_{20}$ = +54,9° (c=0,83, $CH_2Cl_2$);
Analyse für $C_{10}H_{16}O_3$ (184,24): ber.: C 65,2; H 8,75;
gefunden: C 65,4; H 8,5;
$^1$H-NMR (400 MHz) δ = 1,64 (m, 4H, J=5,5 Hz, JO1,5 Hz, H-10, H-4,); 1,78 (m, 2H, H-4, H-6); 1,95 (m, 1H, H-6), 2,15 (m, 2H, H-7); 2,61 (d,d, 1H, J=17 Hz, J=2,7 Hz, H-2ax); 2,72 (d,d, J=17 Hz, J=4,7 Hz, H-2eq); 4,36 (m, 1H, H-3eq); 4,7 (m, 1H, H-5ax); 5,4 (m, 1H, H-8); 5,45 (m, 1H, H-9);
$^{13}$C-NMR (100 MHz) δ = 17,8 (C-10); 27,8 (C-7); 35,4 (C-4); 36,1 (C-6); 38,7 (C-2); 62,7 (C-3); 75,2 (C-5); 126,1 (C-9); 129,75 (C-8); 170,4 (C-1);
IR ($CHCl_3$) m = 3200 - 3600 (OH), 1740 (Lacton)

Beispiel 4

(-)-(3R,5S,8E)-3-Hydroxy-5-dec-8-enolid (Verbindung 1b)

300 mg (1,59 mmol) der Verbindung 3b nach Beispiel 2 werden unter gleichen Bedingungen wie im Beispiel 3 hergestellt.

Das Lacton (Verbindung 1b) ergab 220 mg (75 %) als farbloses Öl.

Rf = 0,49 (Ethylacetat:Hexan/3:1); $[\alpha]^{D}_{20}$ = -25,17° (c=0,146, $CH_2Cl_2$);

Analyse für $C_{10}H_{20}O_3$ (184,24): ber.: C 65,2; H 8,75;
gefunden: C 65,6; H 8,8;

Beispiel 5

(+)-(3R,5R)-3-Hydroxy-5-decanolid (Verbindung 6a)

200 mg (1,1 mmol) der Verbindung 1a nach Beispiel 3 werden in 20 ml Ethylacetat gelöst, mit 30 mg Pd/Aktivkohle versetzt und bei Atmosphärendruck und Raumtemperatur 8 Stunden hydriert. Nach Filtration wird der Katalysator mit 20 ml Ethylacetat gewaschen; die Lösung wird im Vakuum destilliert und der verbleibende Rückstand mit Ethylacetat:Hexan (1:1) an Kieselgel gereinigt. Das farblose ölige Produkt (Verbindung 6a) ergibt 190 mg (94 %).

Rf = 0,54 (Ethylacetat:Hexan/3:1); $[\alpha]^{D}_{20}$ = +35,07° (c=1.5, $CH_2Cl_2$);

Analyse für $C_{10}H_{18}O_3$ (186,25): ber.: C 64,5; H 9,7;
gefunden: C 64,9; H 8,1;

$^1$H-NMR (360 MHz $CDCl_3$) δ = 0,9 (t, 3H, J=6,9 Hz, H-10); 1,2 - 1,74 (m, 4H, 8H, H-6, H-8, H-9); 1,98 (d,d,d,d, 1H, J=14,4 Hz, J=4,0 Hz, J=2,9 Hz, J=1,7 Hz, H-4eq); 2,65 (d,d,d, 1H, J=17,6 Hz, J=3,7 Hz, J=1,7 Hz, H-2eq); 2,72 (d,d, 1H, J=17,6 Hz, J=5,1 Hz, H-2ax); 4,38 (m, 1H, H-3eq); 4,72 (m, 1H, H-5);

$^{13}$C-NMR (90,55 MHz) δ = 13,9 (C-10; 22,5 (C-9); 24,5 (C-7); 31,5 (C-8); 35,5 (C-6); 36,0 (C-4); 38,6 (C-2); 62,8 (C-3); 75,9 (C-5); 170,6 (C-1);

IR ($CHCl_3$) m = 3200 - 3600 (OH), 1740 (Lacton)

Beispiel 6

(-)-(3R,5S)-3-Hydroxy-5-decanolid (Verbindung 6b)

200 mg (1,1 mmol) der Verbindung 1b nach Beispiel 4 werden wie in Beispiel 5 beschrieben hydriert und gereinigt.

Das Lacton (Verbindung 6b) fällt als farbloses Öl an mit einer Ausbeute von 185 mg (92 %).

Rf = 0,53 (Ethylacetat:Hexan/3:1); $[\alpha]^{D}_{20}$ = -39,25° (c=0.91, $CH_2Cl_2$);

Analyse für $C_{10}H_{18}O_3$ (186,25): ber.: C 64,5; H 9,7;
gefunden: C 64,5; H 8,5;

$^1$H-NMR (400 MHz) δ = 0,9,(t, 3H, J=6,9 Hz, H-10); 1,32 (m, 4H, H-8, H-9); 1,39 (m, 1H, H-7); 1,49 (m, 1H, H-7); 1,57 (m, 1H, H-4eq); 1,63 (m, 1H, H-6); 1,73 (m, 1H, H-6); 2,25 (d,d,d,d, 1H, J=13,7 Hz, J=5,5 Hz, J=3,0 Hz, J=1,4 Hz, H-4eq); 2,45 (d,d, 1H, J=17,1 Hz, J=7,9 Hz, H-2ax); 2,89 (d,d,d, 1H, J=17,1 Hz, J=5,9 Hz, J=1,4 Hz, H-2eq); 4,2 (m, 2H, H-3, H-5);

$^{13}$C-NMR (99,55 MHz, $CDCl_3$) δ = 13,9 (C-10); 22,5 (C-9); 24,5 (C-7); 31,5 (C-8); 35,6 (C-6); 37,9 (C-4); 39,5 (C-2); 63,9 (C-3); 77,3 (C-5); 170,7 (C-1)

Beispiel 7

(+)-(2R,4S)-4-Hydroxy-2-methoxy-2-(3E-pentenyl)-tetrahydropyran (Verbindung 4a)

(-)-(2S,4S)-4-Hydroxy-2-methoxy-2-(3E-pentenyl)-tetrahydropyran (Verbindung 4b)

20 g (54 mmol) der Verbindung der Formel IIa, wobei $R^1$ für 2-Penten steht, werden in 500 ml Methanol mit 600 mg $FeCl_3$ 15 min bei Raumtemperatur gerührt. Die Reaktionsmischung wird dann mit Natriumhydrogencarbonatlösung auf pH 7 eingestellt und aufkonzentriert. Chromatographische Trennung an Kieselgel mit Ethylacetat/Hexan/Triethylamin (1:4:0,5) ergibt Verbindung 4a zu 65 % (7 g) und Verbindung 4b zu 5 % (0,538 g).

Verbindung 4a: Rf = 0,36 (Ethylacetat/Hexan 1:2); $[\alpha]^D_{20}$ = +82,6° (c=2.8, $CH_2Cl_2$)
Analyse für $C_{11}H_{20}O_3$ (200,28): ber.: C 66,0; H 10,1;
gefunden: C 65,8; H 10,4;
Verbindung 4b: Rf = 0,60 (Ethylacetat/Hexan 1:2); $[\alpha]^D_{20}$= -66,0° (c=3.0, $CH_2Cl_2$);
Analyse für $C_{11}H_{20}O_3$ (200,2g): ber.: C 66,0; H 10,1;
gefunden: C 66,0; H 10,7;

Beispiel 8

(+)-(2S)-2-Methoxy-2-(3E-pentenyl)-4-tetrahydropyran (Verbindung 5)

7 g (32,7 mmol) der Verbindung 4a aus Beispiel 7 werden mit 800 mg (2,3 mmol) Tetrapropylamoniumruthenat (TPAP) und 8 g (68,3 mmol) N-Methylmorpholin-N-oxid in 200 ml $CH_2Cl_2$ 8 h gerührt. Die Reaktionsmischung wird mit 300 ml $CH_2Cl_2$ verdünnt und zweimal mit 400 ml einer 10 %igen Natriumhydrogensulfitlösung und fünfmal mit Wasser gewaschen. Die organische Phase wird über Natriumsulfat getrocknet, über Celit filtriert und konzentriert. Die Verbindung 5 fällt mit einer Ausbeute von 5,8 g an (89,6 %).
Rf = 0,73 (Ethylacetat:Hexan/3:1); $[\alpha]^D_{20}$ = +75,9° (c=1.3, $CH_2Cl_2$);
Analyse für $C_{11}H_{18}O_3$ (198,26): ber.: C 66,65; H 9,15;
gefunden: C 66,g; H 9,4;

Beispiel 9

(+)-(2S,4R)-4-hydroxy-2-methoxy-2-(3E-pentenyl)-tetrahydropyran (Verbindung 4c)

5.8 g(29,3 mmol) der Verbindung 5 aus Beispiel 8 werden bei -70°C in 350 ml Isopropanol gelöst, 2,2 g (5,2 mmol) Natriumborhydrid zugegeben und bei dieser Temperatur 5 h gerührt. Zur Beseitigung des überschüssigen $NaBH_4$ werden 10 ml über Aluminiumoxid getrocknetes Aceton zugegeben und 15 min bei Raumtemperatur gerührt. Die Mischung wird durch Destillation im Vakuum von Lösemittel entferit; der verbleibende Rückstand wird mit Ethylacetat/Hexan/Triethylamin (1:4:0,5) an Kieselgel gereinigt und ergibt 4,8 g (82 %) Verbindung 4c sowie 0,54 g (9,2 %) Verbindung 4a (siehe Beispiel 7). Wenn LS-Selectride anstatt $NaBH_4$, verwendet wird so entsteht nur 4C.
Verbindung 4c: Rf = 0,60 (Ethylacetat/Hexan 1:2); $[\alpha]^D_{20}$ = +64,5° (c=1.5, $CH_2Cl_2$)
Analyse für $C_{11}H_{20}O_3$ (200,28): ber.: C 66,0; H 10,1;
gefunden: C 66,0; H 10,2;

Beispiel 10

(-)-(3R,8E)-1,3-Dihydroxy-8-decen-5-on (Verbindung 2b)

4,6 g (23,0 mmol) der Verbindung 4c aus Beispiel 9 wird mit 45 mg $FeCl_3$ in 45 ml Isopropanol und 45 ml Wasser gelöst und 30 min bei Raumtemperatur gerührt. Der pH-Wert der Reaktionsmischung wird mit Natriumhydrogencarbonatlösung auf pH 7 bis 8 eingestellt und das Gemisch wird am Rotationsverdampfer zum Sirup aufkonzentriert. Der verbleibende Rückstand wird in 30 ml $CH_2Cl_2$ gelöst und filtriert; Ausbeute 3,9 g (92 %) der Verbindung 2b.
Rf = 0,48 (Ethylacetat/Hexan/Methanol 2:1:0,1); $[\alpha]^D_{20}$ = -23° ((c=1, $CH_2Cl_2$)
Analyse für $C_{10}H_{18}O_3$ (186,25): ber.: C 64,5; H 9,75;
gefunden: C 64,2; H 10,0;
$^1$H-NMR (300 MHz) δ = 1,6 (m, 4H, H-2, H-10); 2,25 (m, 1H, H-7); 2,5 (t, 1H, J=7 Hz, H-6); 2,6 (d, 1H, J=6,5 Hz, H-4); 3,8 (t, 2H, J=5 Hz); 4,3 (m, 1H, H-3); 5,45 (m, 2H, H-8, H-9);
$^{13}$C-NMR (90,55 MHz) δ = 17,7 (C-10); 37,9 (C-2); 43,3 (C-6); 49,3 (C-4); 60,7 (C-1); 67,4 (C-3); 126,1 (C-9); 129,2 (C-8); 211,2 (C-5);

Beispiel 11

(-)-(3R,5S,8E)-1,3,5-Trihydroxy-8-decen (Verbindung 3c)

1 g (5,4 mmol) der Verbindung 2b aus Beispiel 10 wird in 50 ml Tetrahydrofuran mit 8,6 ml einer 1 molaren Triethylboranlösung in Tetrahydrofuran (8,6 mmol) 15 min bei Raumtemperatur gerührt. Nach Abkühlen auf

-70°C werden 4,3 ml Methanol sowie 0,4 g (10,7 mmol) Natriumborhydrid hinzugefügt und die Reaktionsmischung weitere 3 h bei dieser Temperatur gerührt. Zur Aufarbeitung werden 30 ml einer gesättigten Natriumhydrogencarbonatlösung dazugegeben und dreimal mit jeweils 100 ml Essigsäureethylester extrahiert. Die Esterphase wird über Natriumsulfat getrocknet. Nach Abfiltrieren entfernt man das Lösemittel durch Destillation im Vakuum und erhält ein öliges Produkt. Dieses wird noch fünfmal mit Methanol versetzt und im Vakuum destilliert, so daß überschüssiges Boran entfernt wird. Der verbleibende Rückstand wird an Kieselgel mit Aceton:Hexan (2:3) gereinigt und ergibt die Verbindung 3c in einer Ausbeute von 0,84 g (83 %) als ein farbloses Öl.

Rf = 0,40 ($CH_2Cl_2$:$CH_3OH$/9:1); $[\alpha]^D_{20}$ = -4,2° (c=1, $CH_2Cl_2$);

Analyse für $C_{10}H_{20}O_3$ (188,27): ber.: C 63,8; H 10,7;
gefunden: C 63,5; H 10,9;

Die spektroskopischen Daten sind identisch mit denen der Verbindung 3a aus Beispiel 1.

Beispiel 12

(-)-(3R,5R,8E)-1,3,5-Trihydroxy-8-decen (Verbindung 3d)

Zu einer Suspension von 900 mg (23,8 mmol) Natriumborhydrid in 25 ml Tetrahydrofuran werden bei -70°C während 10 min 4,3 ml Eisessig zugetropft und 2 h bei dieser Temperatur gerührt. Nach Zugabe von 1 g (5,4 mmol) der Verbindung 2b aus Beispiel 10 in 5 ml Tetrahydrofuran werden noch 20 ml Eisessig während 30 min zugetropft und die Reaktionsmischung noch 2 h bei dieser Temperatur gerührt.

Nach vollständigem Umsatz bestimmt durch DC wird das Gemisch mit 10 ml Wasser und 30 ml gesättigter Natriumhydrogencarbonatlösung versetzt und dreimal mit je 100 ml Ethylacetat extrahiert. Die organische Phase wird noch dreimal mit je 50 ml Wasser gewaschen und anschließend über Natriumsulfat getrocknet. Nach Entfernen des Lösungsmittels durch Destillation im Vakuum wird der Rückstand an Kieselgel mit Aceton:Hexan (2:3) chromatographisch gereinigt und ergibt die Verbindung 3d mit 76 % (0,78 g) Ausbeute.

Rf = 0,37 ($CH_2Cl_2$:$CH_3OH$/9:1); $[\alpha]^D_{20}$ = -9,2° (c=1, $CH_2Cl_2$);

Analyse für $C_{10}H_{20}O_3$ (188,27): ber.: C 63,8; H 10,7;
gefunden: C 63,4; H 10,3;

Die spektroskopischen Daten sind identisch mit denen der Verbindung 3b aus Beispiel 2.

Beispiel 13

(+)-(3S,5S,8E)-3-hydroxy-5-dec-8-enolid (Verbindung 1c)

300 mg (1,59 mmol) der Verbindung 3c aus Beispiel 11 werden in 15 ml Benzol gelöst und mit 1,39 g (1,59 mmol) Tris-(triphenylphosphin)-ruthenium(II)-chlorid versetzt. Das Gemisch wird 3 Tage bei Raumtemperatur gerührt. Nach Entfernen des Benzols durch Destillation im Vakuum wird der teerige schwarze Rückstand in 2 ml Aceton gelöst und an Kieselgel mit Essigsäureethylester/Hexan (1:1) gereinigt.

Das Lacton (Verbindung 1c) entsteht als farbloses Öl in 72 %iger Ausbeute (210 mg).

Rf = 0,51 (Ethylacetat:Hexan/3:1); $[\alpha]^D_{20}$ = -52,0° (c=0.92, $CH_2Cl_2$);

Analyse für $C_{10}H_{16}O_3$ (184,24): ber.: C 65,2; H 8,75;
gefunden: C 65,6; H 8,3;

Die spektroskopischen Daten sind identisch mit denen der Verbindung aus Beispiel 3.

Beispiel 14

(-)-(3S,5R,8E)-3-Hydroxy-5-dec-8-enolid (Verbindung 1d)

300 mg (1,59 mmol) der Verbindung 3d aus Beispiel 12 werden unter gleichen Bedingungen wie in Beispiel 13 angegeben umgesetzt.

Das erhaltene Lacton (Verbindung 1d) ergab 210 mg (73 %) Ausbeute als farbloses Öl.

Rf = 0,49 (Ethylacetat:Hexan/3:1); $[\alpha]^D_{20}$ = +25,5° (c=0,14 $CH_2Cl_2$);

Analyse für $C_{10}H_{20}O_3$ (184,24): ber.: C 65,2; H 8,75;
gefunden: C 65,6; H 8,3;

Die spektroskopischen Daten sind identisch mit denen der Verbindung 1b aus Beispiel 4

Beispiel 15

(+)-(3S,5S)-3-Hydroxy-5-decanolid (Verbindung 6c)

200 mg (1,1 mmol) der Verbindung 1c aus Beispiel 13 weiden in 20 ml Ethylacetat gelöst, mit 30 mg Pd/Aktivkohle versetzt und bei Atmosphärendruck und Raumtemperatur 8 Stunden hydriert. Nach Filtration wird der Katalysator mit 20 ml Ethylacetat gewaschen; die Lösung wird im Vakuum destilliert und der verbleibende Rückstand mit . Ethylacetat:Hexan (1:1) an Kieselgel gereinigt. Das farblose ölige Produkt (Verbindung 6c) ergibt 180 mg (92 %) Ausbeute.

Rf = 0,54 (Ethylacetat:Hexan/3:1); $[\alpha]^{D}_{20}$ = -34,1° (c=1.5, $CH_2Cl_2$);

Analyse für $C_{10}H_{18}O_3$ (186,2,5): ber.: C 64,5; H 9,7;
gefunden: C 64,8; H 8,9;

Die spektroskopischen Daten sind identisch mit denen der Verbindung 6a aus Beispiel 5.

Beispiel 16

(-)-(3S,5R)-3-Hydroxy-5-decanolid (Verbindung 6d)

200 mg (1,1 mmol) der Verbindung 1d aus Beispiel 14 werden wie in Beispiel 15 beschrieben hydriert und gereinigt.

Das Lacton (Verbindung 6d) fällt als farbloses Öl an und ergibt eine Ausbeute von 185 mg (92 %).

Rf = 0,53 (Ethylacetat:Hexan/3:1); $[\alpha]^{D}_{20}$ = +40,1° (c=1.0, $CH_2Cl_2$);

Analyse für $C_{10}H_{18}O_3$ (186,25): ber.: C 64,5; H 9,7;
gefunden: C 64,5; H 8,9;

Die spektroskopischen Daten sind identisch mit denen der Verbindung 6b aus Beispiel 6.

Beispiel 17

(3S,5S,6E,8E)-1,3,5-Trihydroxy-6,8-decadien (Verbindung 3e)

1 g (5,4 mol) der Verbindung der Formel IIa, wobei $R^1$ für 2,4-Dipenten steht, wird wie in Beispiel 1 angegeben umgesetzt.

Analyse für $C_{10}H_{18}O_3$ (186,27): ber.: C 64,49; H 9,79
gefunden: C 64,1 ; H 9,7

$^1$H-NMR (400 MHz) δ = 1,5-2,0 m; 3,9 (m, 2H, H-1); 4,25 (m, 1H, H-3); 4,55 (m, 1H, H-5); 5,6-5,7 (m, 3H); 6,1 (m, 1H); 6,25 (m, 1H)

Beispiel 18

(3S,5R,6E,8E)-3-Hydroxy-5-deca-6,8-dienolid (Verbindung 1e)

300 mg der Verbindung 3e nach Beispiel 17 werden wie in Beispiel 3 angegeben umgesetzt.

Analyse für $C_{10}H_{14}O_3$ (182,22): ber.: C 65,92; H 7,74
gefunden: C 65,8 ; H 7,6

$^{13}$C-NMR (100 MHz) δ = 18,0 (C-1); 36,4 (C-9); 38,7 (C-2); 62,55 (C-3); 76,0 (C-5); 127,0; 130,1; 131,8; 133,0 (C-H); 170,15 (C-1)

Beispiel 19

(3R,5S)-3,6-Dihydroxy-5-hexanolid (Verbindung 1f)

1 g der Verbindung 1e nach Beispiel 18 werden in 30 ml Methanol gelöst, mit 80 mg Natriumhydrid versetzt und bei 25°C für 5 Stunden gerührt. Es wird mit 2N HCl neutralisiert, einrotiert und in 30 ml Aceton gelöst. Die Lösung wird mit etwas $ZnCl_2$ versetzt und am Rückfluß für 12 Stunden gekocht. Anschließend wird mit einer Mischung von Wasser und Ethylacetat (1:1, je 100 ml) extrahiert. Die Ethylacetat-Phase wird über Natriumsulfat getrocknet und nach dem Einrotieren in 150 ml Methanol aufgenommen. Die Ozonolyse erfolgt bei -70°C (Organikum, 14. Auflage, 1975, Seite 295; VEB-Verlag). Anschließend wird 1 g $NaBH_4$ zugegeben und für 2 Stunden gerührt.

Danach wird einrotiert und mit einer Mischung aus Wasser und Methylenchlorid (1:1; je 150 ml) extrahiert. Anschließend wird einrotiert und das Konzentrat auf einer Kieselgelsäule (Essigester/Hexan, 1:10 auf 10:1) gereinigt.

Es werden 150 mg von (3R,5S)-3,5-O-Isopropyliden-3,5,6-trihydroxyhexansäuremethylester erhalten.

Analyse für $C_{10}H_{18}O_5$ (218,25): ber.: C 55,03; H 8,31
gefunden: C 55,1; H 8,2

Mit Trifluoressigsäure in 15 äquivalenten Dichlormethan wird (3R,5S)-3,5-O-Isopropyliden-3,5,6-trihydroxyhexansäuremethylester in (3R,5S)-3,6-Dihydroxy-5-hexanolid, bei 23°C über-24 Stunden umgesetzt (US 4,970,313).

Beispiel 20

Monolayer von HEP-G2-Zellen in lipoproteinfreiem Nährmedium werden mit entsprechenden Konzentrationen der zu prüfenden Substanzen aus Beispiel 3 und 5 eine Stunde vorinkubiert. Nach Zugabe der $^{14}$C-markierten Biosynthesevorstufe [$^{14}$C]Natriumacetat wird die Inkubation für 3 Stunden fortgesetzt. Danach wird ein Teil der Zellen alkalisch verseift, bei vorheriger Zugabe eines internen Standards von $^{3}$H-Cholesterin. Die Lipide der verseiften Zellen werden mit einem Gemisch aus Chloroform-Methanol extrahiert. Dieses Lipidgemisch wird nach Zusatz von Trägercholesterin präparativ dünnschicht-chromatographisch aufgetrennt, die Cholesterinbande nach Anfärbung isoliert und die aus dem $^{14}$C-Precursor gebildete Menge $^{14}$C-Cholesterin szintigraphisch bestimmt. In einem aliquoten Teil der Zellen wurde Zellprotein bestimmt, sodaß die in der Zelleinheit pro mg Zellprotein aus $^{14}$C-Vorläufer gebildete Menge $^{14}$C-Cholesterins berechnet werden kann. Zum Vergleich für die Hemmwirkung eines zugesetzten Prüfpräparates dient die Kontrolle, sodaß direkt die Hemmung der Cholesterinbiosynthese bei einer bestimmten molaren Konzentration des Prüfpräparates im Medium angegeben werden kann. In aliquoten Anteilen der Zellkultur wird die Intaktheit der Zellkultur und die fehlende Zellschädigung durch Präparateeinwirkung morphologisch (Lichtmikroskopie) beurteilt und biochemisch durch Bestimmung der Laktat-Dehydrogenase Ausschüttung in Inkubationsmedium gemessen. Als Standartpräparat wurde Lovostatin benutzt. Die Ergebnisse werden in Tabelle 1 gezeigt.

Tabelle 1

| Verbindung | Konzentration [M] | Cholesterin in Prozent von der Kontrolle |
|---|---|---|
| 1a | $10^{-5}$ | 56 |
| 1a | $10^{-7}$ | 72 |
| 6a | $10^{-5}$ | 45 |
| 6a | $10^{-7}$ | 61 |
| 3e | $10^{-6}$ | 30 |
| 1e | $10^{-6}$ | 30 |

**Patentansprüche**

1. Verfahren zur Herstellung von 5-substituierten 3R5R, 3R5S, 3S5S und 3S5R δ-Lactonen der Formel Ia, Ib, Ic und Id,

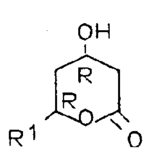 (Ia) 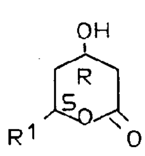 (Ib) 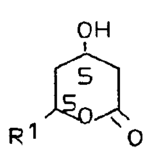 (Ic) 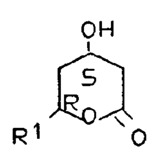 (Id)

wobei $R^1$ für

1) geradkettiges oder verzweigtes Alkyl mit 3 bis 15 C-Atomen,
2) geradkettiges oder verzweigtes Alkenyl mit 3 bis 15 C-Atomen und 1 bis 7 C-C-Doppelbindungen, oder
3) $CH_2OH$ steht, dadurch gekennzeichnet, daß man

a) eine Verbindung der Formel IIa

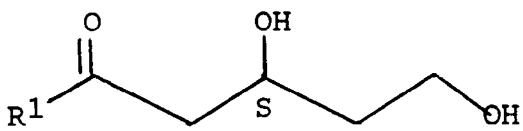

(IIa)

1) zu einer Verbindung der Formel IIIa und IIIb,

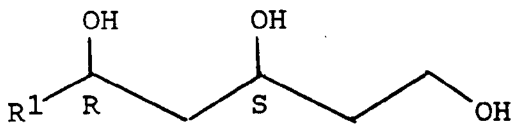

(IIIa)

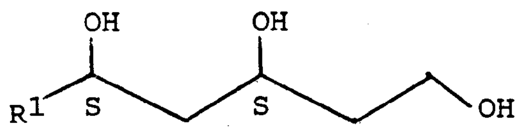

(IIIb)

diastereoselektiv reduziert,
2) zu einer Verbindung der Formel Ia und Ib,

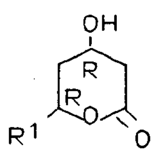

(Ia)

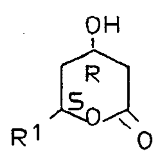

(Ib)

regioselektiv oxidiert;
und gegebenenfalls
3) die Doppelbindungen der Alkylenkette hydriert, oder

b) die Verbindung der Formel IIa zu
1) einer Verbindung der Formel IVa und IVb,

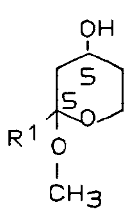

(IVa)

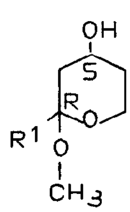

(IVb)

acetalisiert,
2) zu einer Verbindung der Formel V,

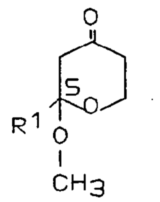

(V)

oxidiert,

3) zu einer Verbindung der Formel IVc,

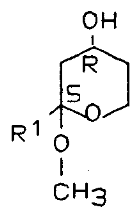

(IVc)

diastereoselektiv reduziert,

4) zu einer Verbindung der Formel IIb,

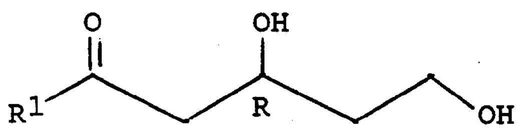

(IIb)

deacetalisiert,

5) zu einer Verbindung der Formel IIIc und IIId,

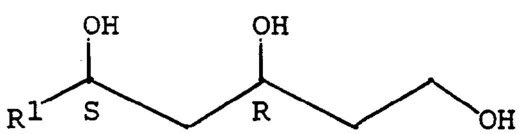

(IIIc)

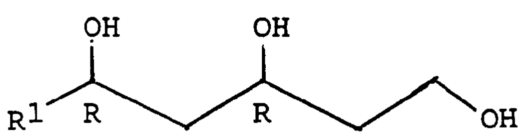

(IIId)

diastereoselektiv reduziert,

6) zu einer Verbindung der Formel Ic und Id,

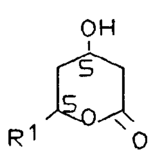

(Ic)

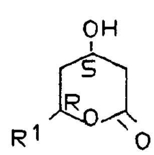

(Id)

regioselektiv oxidiert und gegebenenfalls

7) die Doppelbindungen in der Alkylenkette hydriert oder

c) die Verbindung der Formel Ia, Ib, Ic oder Id, wobei $R^1$ für ,

1) geradkettiges oder verzweigtes Alkyl mit 3 bis 15 C-Atomen

2) geradkettiges oder verzweigtes Alkenyl mit 3 bis 15 C-Atomen und 1 bis 7 C-C-Doppelbindungen steht,

1) mit einer Verbindung der Formel $R^3$-$O^-$ zu einer Verbindung der Formel

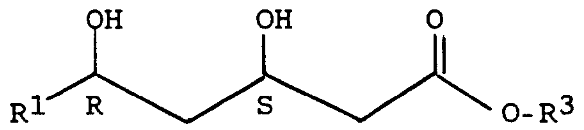

(Va)

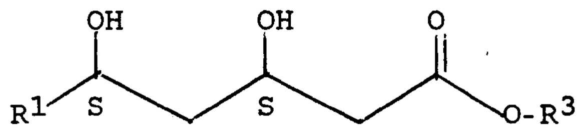

(Vb)

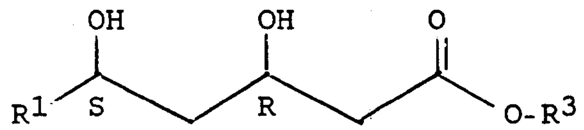

(Vc)

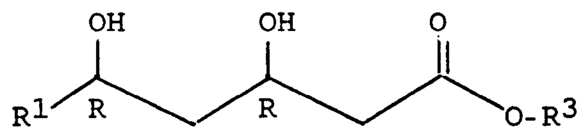

(Vd)

umsetzt, wobei $R^3$ für Alkyl mit 1 bis 5 C-Atomen steht,

2) mit Aceton oder Dimethoxypropan zu einer Verbindung der Formeln VIa, VIb, VIc oder VId ketalisiert,

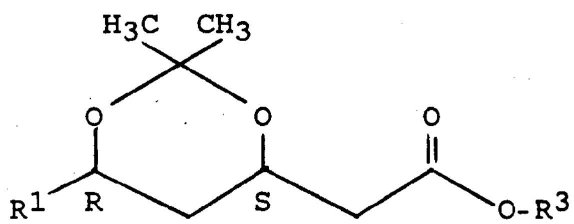

(VIa)

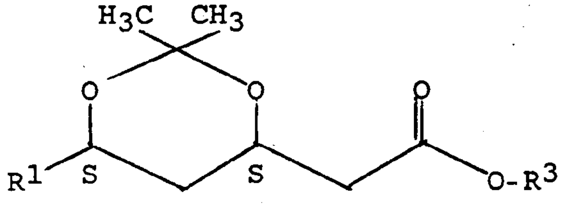

(VIb)

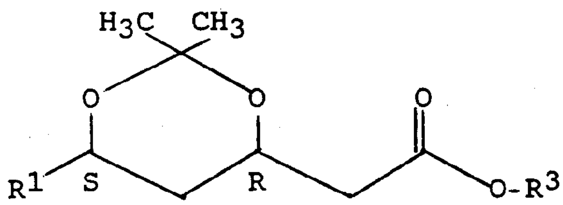

(VIc)

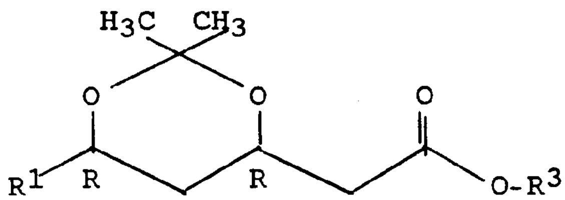

(VId)

3) mit Ozon zu einer Verbindung der Formeln VIIa, VIIb, VIIc oder VIId,

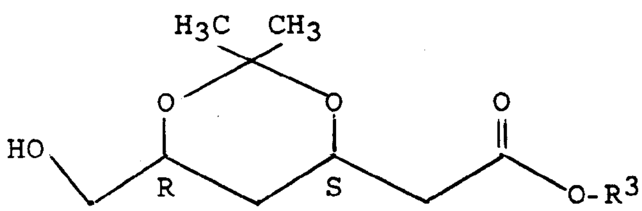

(VIIa)

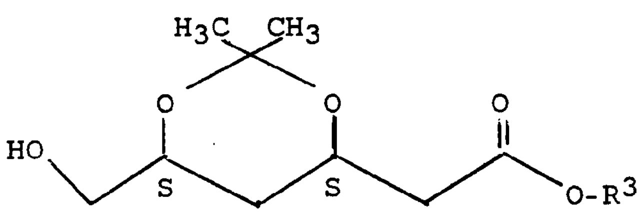

(VIIb)

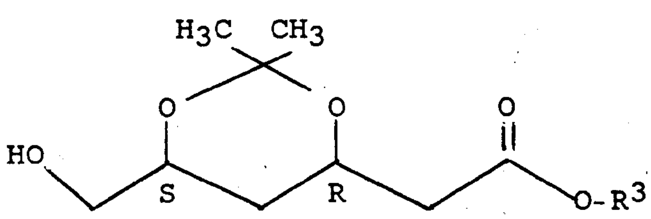

(VIIc)

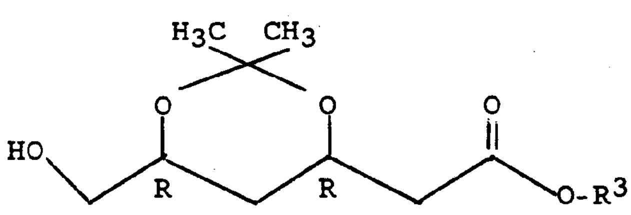

(VIId)

oxidiert und

4) zu einer Verbindung der Formeln Ia, Ib, Ic oder Id umsetzt wobei $R^1$ für $CH_2OH$ steht.

2. Verfahren nach Anspruch 1, dadurch gekennzeichnet, daß $R^1$ für
   1) geradkettiges oder verzweigtes Alkyl mit 3 bis 10 C-Atomen,
   2) geradkettiges oder verzweigtes Alkenyl mit 3 bis 10 C-Atomen und 1 bis 5 C-C-Doppelbindungen steht.

3. Verfahren nach Anspruch 1, dadurch gekennzeichnet, daß $R^1$ für
   1) geradkettiges oder verzweigtes Alkyl mit 5 C-Atomen,
   2) geradkettiges oder verzweigtes Alkenyl mit 5 C-Atomen und 1 oder 2 C-C-Doppelbindungen steht.

4. Verbindung der Formel Ia, Ib, Ic und Id

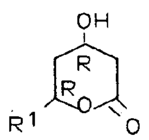 (Ia) 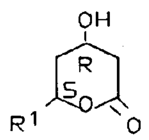 (Ib) 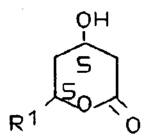 (Ic) 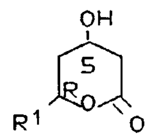 (Id)

   wobei $R^1$ für
   1) geradkettiges oder verzweigtes Alkyl mit 3 bis 15 C-Atomen,
   2) geradkettiges oder verzweigtes Alkenyl mit 3 bis 15 C-Atomen und 1 bis 7 C-C-Doppelbindungen, oder
   3) $CH_2OH$ steht,
   ausgenommen die Verbindung der Formel Ia, wobei $R^1$ für eine geradkettige Alkylkette mit 5 C-Atomen steht.

5. Verbindung der Formel Ia, Ib, Ic und Id nach Anspruch 4, dadurch gekennzeichnet, daß $R^1$ für
   1) geradkettiges oder verzweigtes Alkyl mit 3 bis 10 C-Atomen,
   2) geradkettiges oder verzweigtes Alkenyl mit 3 bis 10 C-Atomen und 1 bis 5 C-C-Doppelbindungen steht.

6. Verbindung der Formel Ia, Ib, Ic und Id nach anspruch 1, dadurch gekennzeichnet, daß $R^1$ für
   1) geradkettiges oder verzweigtes alkyl mit 5 C-atomen,
   2) geradkettiges oder verzweigtes alkenyl mit 5 C-Atomen und 1 oder 2 C-C-Doppelbindungen steht.

7. Arzneimittel, gekennzeichnet durch einen wirksamen Gehalt an mindestens einer Verbindung der Formel Ia, Ib, Ic oder Id nach einem oder mehreren der ansprüche 4 bis 6 oder mindestens einer nach dem Verfahren gemäß einem oder mehreren der ansprüche 1 bis 3 hergestellten Verbindung, neben einem physiologisch annehmbaren Träger und gegebenenfalls weiteren Zusatz und/oder Hilfsstoffen, ausgenommen die Verbindung der Formel Ia nach anspruch 4, wobei $R^1$ für eine geradkettige Alkylkette mit 5 C-Atomen steht.

8. Verfahren zur Herstellung eines Arzneimittels gemäß Anspruch 7, dadurch gekennzeichnet, daß man mindestens eine Verbindung der Formel Ia, Ib, Ic oder Id gemäß einem oder mehreren der Ansprüche 4 bis 6 oder wie erhalten nach einem oder mehreren der Ansprüche 1 bis 3, ausgenommen die Verbindung der Formel Ia nach Anspruch 1, wobei $R^1$ für eine geradkettige Alkylkette mit 5 C-Atomen steht, mit einem physiologisch annehmbaren Träger und gegebenenfalls weiteren Zusatz und/oder Hilfsstoffen in eine geeignete Darreichungsform bringt.

9. Verwendung von mindestens einer Verbindung der Formel Ia

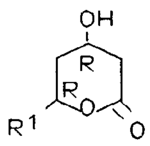

(Ia)

wobei $R^1$ für

1) geradkettiges oder verzweigtes Alkyl mit 3 bis 15 C-Atomen,

2) geradkettiges oder verzweigtes Alkenyl mit 3 bis 15 C-Atomen und 1 bis 7 C-C-Doppelbindungen oder

3) $CH_2OH$ steht,

neben einem physiologisch annehmbaren Träger und gegebenenfalls weiteren Zusatz- und/oder Hilfsstoffen zur Herstellung eines Arzneimittels mit Cholesterinsynthese hemmender Wirkung.

**10.** Verbindung der Formel IIb, IIIa, IIIc und IIId,

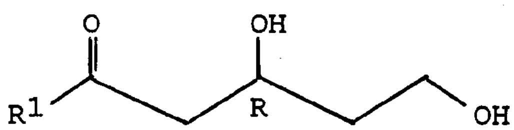

(IIb)

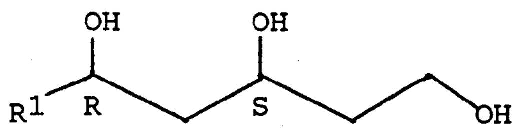

(IIIa)

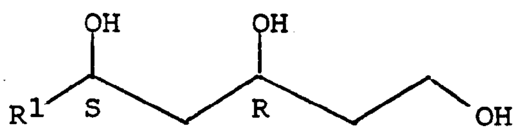

(IIIc)

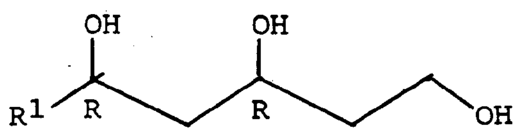

(IIId)

wobei $R^1$ die in Anspruch 4 genannte Bedeutung hat.

**11.** Verfahren zur Herstellung der Verbindung der Formel IIIa, IIIb, IIIc und IIId gemäßt Anspruch 1, dadurch gekennzeichnet, daß man

a) eine Verbindung der Formel IIb

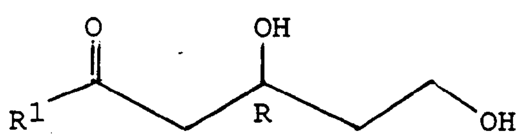

(IIb)

zu einer Verbindung der Formel IIIc oder IIId diasteroselektiv reduziert oder

b) die Verbindung der Formel IIa

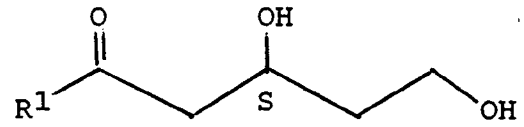

(IIa)

zu der Verbindung der Formel IIIa oder IIIb diastereoselektiv reduziert.

**12.** Verfahren zur Herstellung der Verbindung IIb gemäß Anspruch 10, dadurch gekennzeichnet, daß man die Verbindung der Formel IVc gemäß Anspruch 1 deacetalisiert.

**Patentansprüche fur fölgende Vertragsstaaten : ES, GR**

**1.** Verfahren,zur Herstellung von 5-substituierten 3R5R, 3R5S, 3S5S und 3S5R δ-Lactonen der Formel Ia, Ib, Ic und Id,

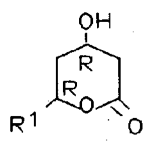 (Ia) 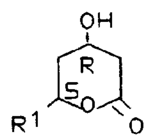 (Ib) 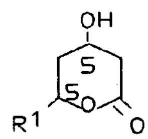 (Ic) 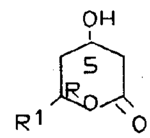 (Id)

wobei $R^1$ für

1) geradkettiges oder verzweigtes Alkyl mit 3 bis 15 C-Atomen,
2) geradkettiges oder verzweigtes Alkenyl mit 3 bis 15 C-Atomen und 1 bis 7 C-C-Doppelbindungen, oder
3) $CH_2OH$ steht, dadurch gekennzeichnet, daß man
a) eine Verbindung der Formel IIa

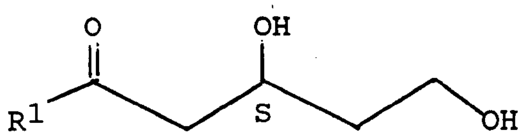

(IIa)

1) zu einei Verbindung der Formel IIIa und IIIb,

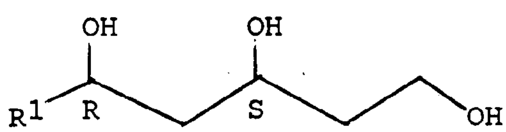

(IIIa)

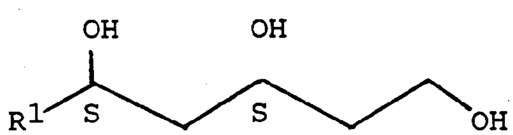

(IIIb)

diastereoselektiv reduziert,
2) zu einer Verbindung der Formel Ia und Ib,

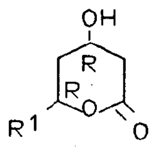 (Ia) 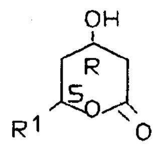 (Ib)

regioselektiv oxidiert,
und gegebenenfalls
3) die Doppelbindungen der Alkylenkette hydriert, oder
b) die Verbindung der Formel IIa zu
1) einer Verbindung der Formel IVa und IVb,

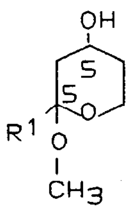 (IVa) 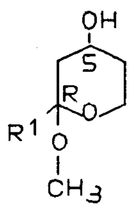 (IVb)

acetalisiert,
2) zu einer Verbindung der Formel V,

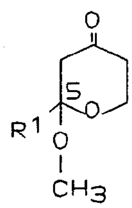 (V)

oxidiert,
3) zu einer Verbindung der Formel IVc,

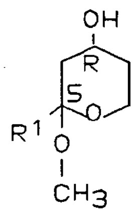 (IVc)

diastereoselektiv reduziert,
4) zu einer Verbindung der Formel IIb,

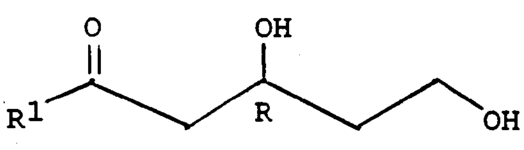 (IIb)

deacetalisiert,
5) zu einer Verbindung der Formel IIIc und IIId,

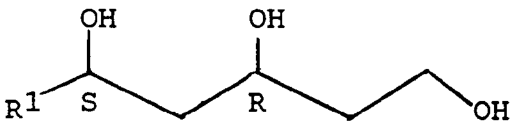

(IIIc)

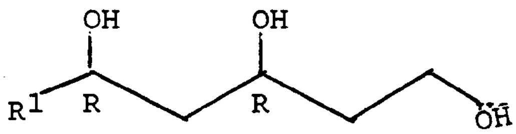

(IIId)

diastereoselektiv reduziert,
6) zu einer Verbindung der Formel Ic und Id,

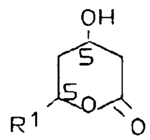

(Ic)

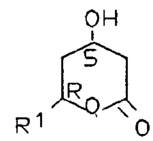

(Id)

regioselektiv oxidiert und gegebenenfalls
7) die Doppelbindungen in der Alkylenkette hydriert oder
c) die Verbindung der Formel Ia, Ib, Ic oder Id, wobei $R^1$ für
1) geradkettiges oder verzweigtes Alkyl mit 3 bis 15 C-Atomen
2) geradkettiges oder verzweigtes Alkenyl mit 3 bis 15 C-Atomen und 1 bis 7 C-C-Doppelbindungen steht,
1) mit einer Verbindung der Formel $R^3$-$O^-$ zu einer Verbindung der Formel

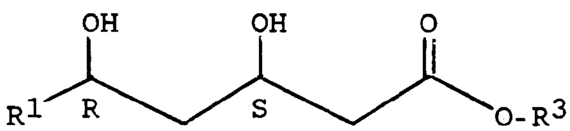

(Va)

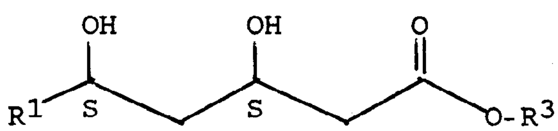

(Vb)

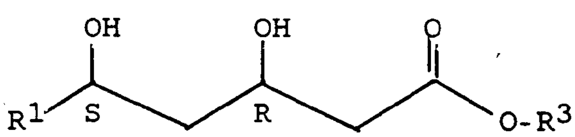

(Vc)

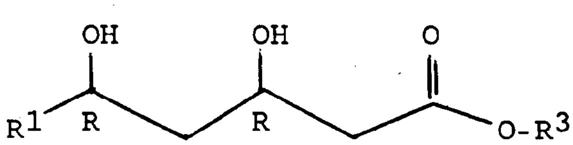

(Vd)

umsetzt, wobei $R^3$ für Alkyl mit 1 bis 5 C-Atomen steht,
2) mit Aceton oder Dimethoxypropan zu einer Verbindung der Formeln VIa, VIb, VIc oder VId ketalisiert,

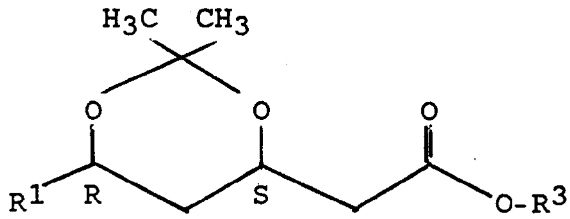

(VIa)

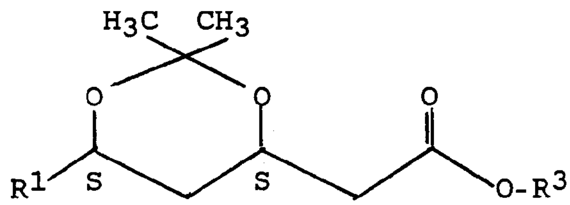

(VIb)

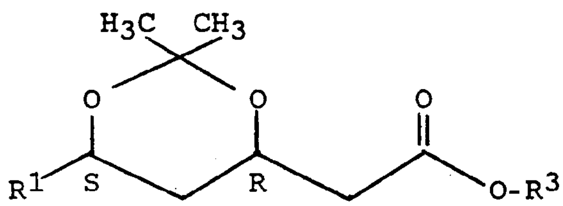

(VIc)

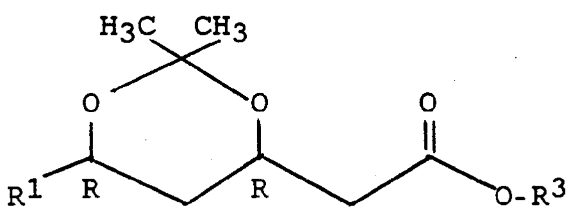

(VId)

3) mit Ozon zu einer Verbindung der Formeln VIIa, VIIb, VIIc oder VIId,

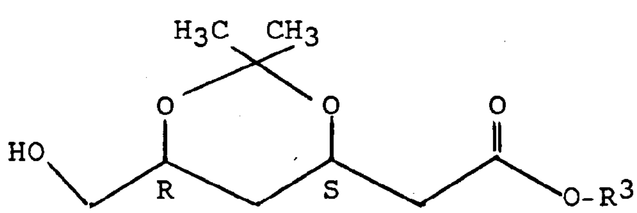

(VIIa)

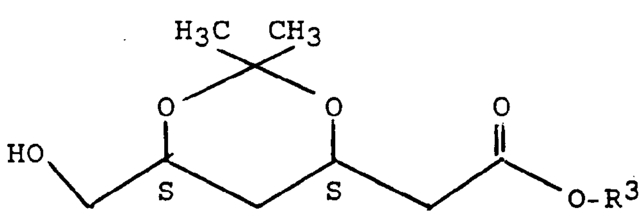

(VIIb)

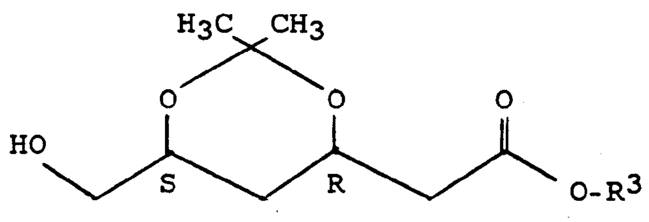

(VIIc)

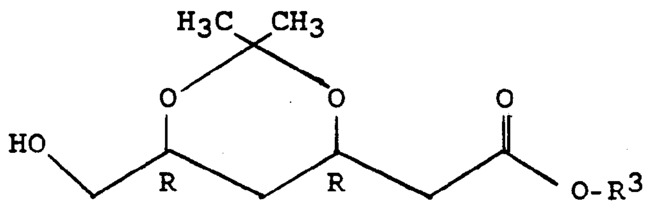

(VIId)

oxidiert und

4) zu einer Verbindung der Formeln Ia, Ib, Ic oder Id umsetzt wobei $R^1$ für $CH_2OH$ steht.

**2.** Verfahren nach Anspruch 1, dadurch gekennzeichnet, daß $R^1$ für
1) geradkettiges oder verzweigtes Alkyl mit 3 bis 10 C-Atomen,
2) geradkettiges oder verzweigtes Alkenyl mit 3 bis 10 C-Atomen und 1 bis 5 C-C-Doppelbindungen steht.

**3.** Verfahren nach Anspruch 1, dadurch gekennzeichnet, daß $R^1$ für
1) geradkettiges oder verzweigtes Alkyl mit 5 C-Atomen,
2) geradkettiges oder verzweigtes Alkenyl mit 5 C-Atomen und 1 oder 2 C-C-Doppelbindungen steht.

**4.** Verfahren zur Herstellung eines Arzneimittels, dadurch gekennzeichnet, daß man mindestens eine Verbindung der Formel Ia, Ib, Ic oder Id erhältlich nach einem oder mehreren der Ansprüche 1 bis 3, ausgenommen die Verbindung der Formel Ia nach Anspruch 1, wobei $R^1$ für eine geradkettige Alkylkette mit 5 C-Atomen steht, mit einem physiologisch annehmbaren Träger und gegebenenfalls weiteren Zusatz und/oder Hilfsstoffen in eine geeignete Darreichungsform bringt.

**5.** Verwendung von mindestens einer Verbindung der Formel Ia

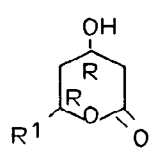

(Ia)

wobei $R^1$ für
1) geradkettiges oder verzweigtes Alkyl mit 3 bis 15 C-Atomen,
2) geradkettiges oder verzweigtes Alkenyl mit 3 bis 15 C-Atomen und 1 bis 7 C-C-Doppelbindungen, oder
3) $CH_2OH$ steht,

neben einem physiologisch annehmbaren Träger und gegebenenfalls weiteren Zusatz- und/oder Hilfsstoffen zur Herstellung eines Arzneimittels mit Cholesterinsynthese hemmender Wirkung.

**6.** Verbindung der Formel IIb, IIIa, IIIc ünd IIId,

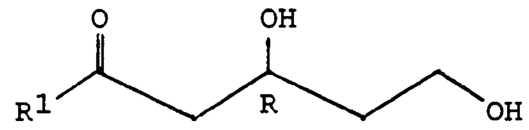

(IIb)

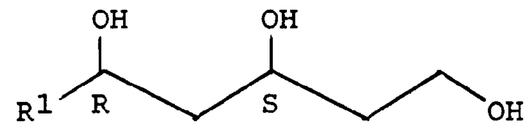

(IIIa)

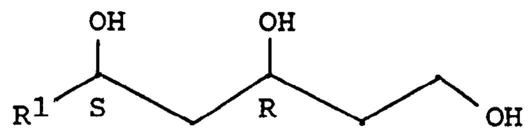

(IIIc)

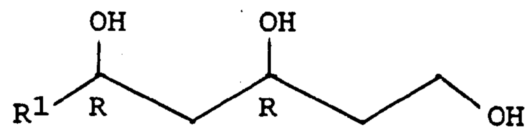

(IIId)

wobei $R^1$ die in Anspruch 1 genannte Bedeutung hat.

7. Verfahren zur Herstellung der Verbindung der Formel IIIa, IIIb, IIIc und IIId gemäßt Anspruch 1, dadurch gekennzeichnet, daß man

a) eine Verbindung der Formel IIb

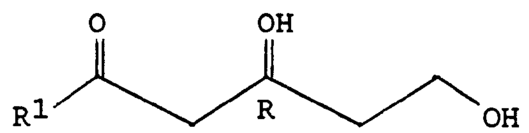

(IIb)

zu einer Verbindung der Formel IIIc oder IIId diasteroselektiv reduziert oder

b) die Verbindung der Formel IIa

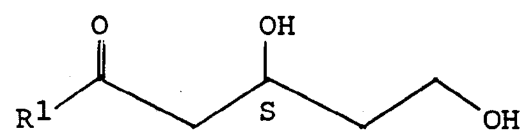

(IIa)

zu der Verbindung der Formel IIIa oder IIIb diastereoselektiv reduziert.

8. Verfahren zur Herstellung der Verbindung IIb gemäß Anspruch 6, dadurch gekennzeichnet, daß man die Verbindung der Formel IVc gemäß Anspruch 1 deacetalisiert.